# EUROPEAN PATENT SPECIFICATION

(11) **EP 3 150 154 B1**
(45) Date of publication and mention of the grant of the patent: **30.10.2019**
(21) Application number: 15425074.0
(22) Date of filing: 29.09.2015
(51) Int. Cl.: A61B 17/86, A61B 17/74, A61B 17/88

(54) **ENDOSSEOUS SCREW ASSEMBLY AND INTERNAL FIXATION SYSTEM COMPRISING SAID ENDOSSEOUS SCREW ASSEMBLY**
ENOSSALE SCHRAUBENANORDNUNG UND INTERNES FIXATIONSSYSTEM MIT DIESER ENOSSALEN SCHRAUBENANORDNUNG
ENSEMBLE VIS ENDO-OSSEUSES ET SYSTÈME DE FIXATION INTERNE COMPRENANT LEDIT ENSEMBLE

(43) Date of publication of application: 05.04.2017
(73) Proprietor: Orthofix S.r.l., 37012 Bussolengo (VR) (IT)
(72) Inventor: LORENZINI, Denis, I-37013 Caprino Veronese (Verona) (IT); VICENZI, Federico, I-38024 Peio (Trento) (IT)
(74) Representative: Ferreccio, Rinaldo

(56) References cited:
- WO-A2-2004/110292
- WO-A2-2007/124099
- US-A1- 2007 270 848
- US-A1- 2014 214 098

## Description

### Field of application

The present invention is applicable to the field of orthopaedic surgery and relates to an endosseous screw assembly, in particular of the type used in internal fixation systems comprising plates or nails.

The invention also relates to the internal fixation system comprising said endosseous screw assembly.

### Prior art

The internal fixation systems, which are widely used in the orthopaedics sector, embrace different types of bone implants which are generally applied in order to stabilize the fractured bone site of a patient.

The internal fixation systems may comprise a bone plate, which is fixed in contact with an external surface of the fractured bone in order to ensure alignment and fixing of two or more segments thereof. In order to allow fixing of the plate, the system envisages in these cases a plurality of bone screws which pass through a corresponding number of holes formed in the element.

The internal fixation systems may also comprise an endomedullary nail which is typically inserted inside the medullary canal of the long bone of a patient. In this case also, generally one or more bone screws are provided, these passing through the bone cortex in the transverse direction and interfacing with the endomedullary nail in order to stabilize the system.

In both types of implant described above, it is required to provide a stable connection between the stem of the screw and the body of the plate or the nail. This connection must be able to transmit onto the plate/nail torsional and flexional stresses which are applied to the screw stem, allowing at the same time controlled axial sliding of said stem so as to allow the screwing and removal operations.

In order to satisfy the aforementioned requirements, the connection systems known in the art are generally relatively elaborate and complex, so that they constitute a critical phase of the fixation system implant both from the point of view of time and as regards the attention required by the surgeon.

For example, WO 2007/124099 A2 discloses an intramedullary osteosynthetic device including an intramedullary nail, a hip helical implant, a sliding sleeve, a lateral set screw, and at least one distal locking screw. The hip helical implant includes at least two helical twisted blades attached to the frontal helical portion, each blade of the hip helical implant has a hole for receiving a peg from a step helix fixating the hip helical implant to the step helix.

In particular, in the case of a fracture in the tranchanter zone, common practice requires stabilization of the fracture using one or more screws in the neck of the femur and several screws in the middle part of the femur. The screw placed in the neck of the femur usually is large in size and must be able to slide along its axis.

Therefore, the implant of the device requires the surgeon to carry out several consecutive operations aimed at ensuring the correct positioning of the screw inside the femoral neck.

Basically, the devices which are currently widely used require particular attention during insertion of the screw which is performed by means of a special tool, the orientation of which determines the correct insertion thereof into the patient's bone.

Consequently, the surgeon is required to carry out different preliminary operations before performing insertion of the screw.

Moreover, the connection of the screw to the plate/nail requires generally, after the aforementioned screw has been implanted, the insertion of a further locking element.

Such a surgical procedure must therefore be carried out with particular attention and unfortunately may not be assigned to surgeons with little experience, despite the fact that such operations are now considered to be routine in the orthopaedics departments of hospitals.

Moreover it is clear how this increases the duration of the operations, with greater risks for the health of patients, in particular if they are elderly.

The technical problem underlying the present invention is therefore that of providing an endosseous screw assembly and associated internal fixation system which allow a reduction in the duration of the surgical operations and which may be implanted by means of a limited number of operations carried out by the surgeon; at the same time it is also desirable that the operational methods should be those which are already known to the surgeon so as to be easily applied also by less expert surgeons.

### Summary of the invention

The aforementioned technical problem is solved by an endosseous screw assembly according to claim 1 as well as an internal fixation system comprising said assembly, according to claim 10, and an installation kit comprising said fixation system, according to claim 14.

The dependent claims describe preferred and particularly advantageous embodiments, in accordance with the present invention.

Further characteristic features and advantages will emerge more clearly from the detailed description provided hereinbelow of a preferred, but not exclusive embodiment of the present invention, with reference to the attached figures, provided by way of a non-limiting example.

### Brief description of the drawings

Figure 1 shows a perspective view of an internal fixation system comprising an endosseous screw assembly according to the present invention;
Figure 2 is a cross-sectional view of Figure 1;
Figure 3 shows a view, on a larger scale, of only the nail of Figure 1;
Figure 4 shows a perspective view of the endosseous screw assembly according to Figure 1;
Figure 5 is a cross-sectional view of Figure 4;
Figure 6 shows the view of Figure 4 in which only the ring nut is shown cross-sectioned;
Figure 7 shows a view, from a different perspective, of the system according to Figure 1;
Figure 8 shows a view, on a larger scale, of Figure 7;
Figure 9 shows a perspective view of the internal fixation system according to Figure 1 connected to a tool for fixing the endosseous screw assembly;
Figure 10 is a cross-sectional view of Figure 9 without the bone nail;

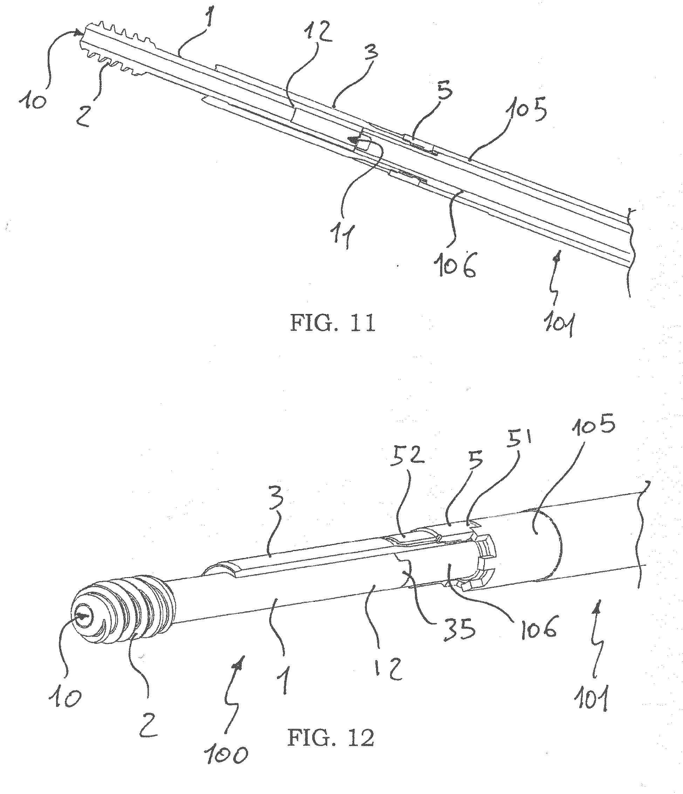

Figure 11 shows a view, on a larger scale, of Figure 10;
Figure 12 shows a detail of the engaging system, with the endosseous screw assembly partly cross-sectioned;
Figure 13 shows a further view, on a larger scale, of Figure 10;
Figure 14 shows a view, on a larger scale, of Figure 12.

### Detailed description

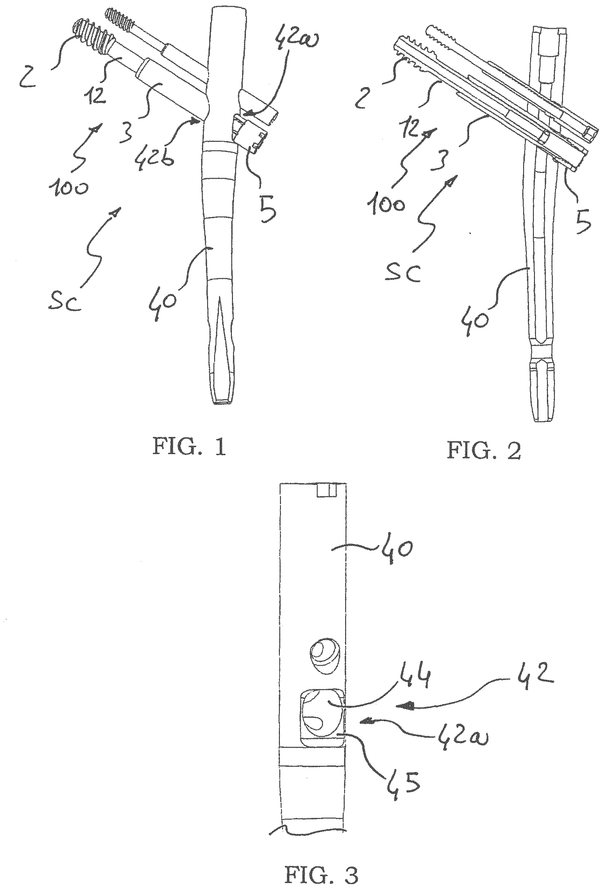

With reference to the attached figures, and in particular Figures 1, 2 and 7, the reference letters SC indicates an internal fixation system in accordance with an embodiment of the present invention.

In this embodiment, the internal fixation system SC comprises an endomedullary nail 40, in particular a trochanteric nail, and an endosseous screw assembly 100.

The endosseous screw assembly 100 is inserted and locked onto the trochanteric nail 40, inclined with respect to the axis of the nail, in such a way that it is anchored inside the acetabular head of the patient.

In an alternative embodiment, not shown, the fixation system may be a bone plate fixed to the femoral surface of a patient by means of an endosseous screw assembly 100 in accordance with the present invention.

Essentially, both the endomedullary nail and the bone plate may be used as internal fixation member, using the same endosseous screw assembly 100.

The internal fixation member 40 has at least one connection hole 42 for locking the endosseous screw assembly 100.

The connection hole 42 has an entry side 42a and an exit side 42b for the endosseous screw assembly, which are connected by means of an internal wall 44 and aligned along an axis which defines the inclination of the endosseous screw assembly with respect to the fixation member.

The internal wall 44 of the connection hole, which is substantially circular, defines the internal diameter of the hole.

The entry side 42a has a flattened part which defines a flat surface 45 lying parallel to the longitudinal axis of the fixation system, in the region of the connection hole.

This flat surface 45 is formed in the vicinity of the perimeter of the connection hole.

It should be noted that the fixation member 40 may comprise one or more connection holes, so as to allow insertion of a variable number of endosseous screw assemblies, depending on the clinical conditions of the patient, again within the context the present invention.

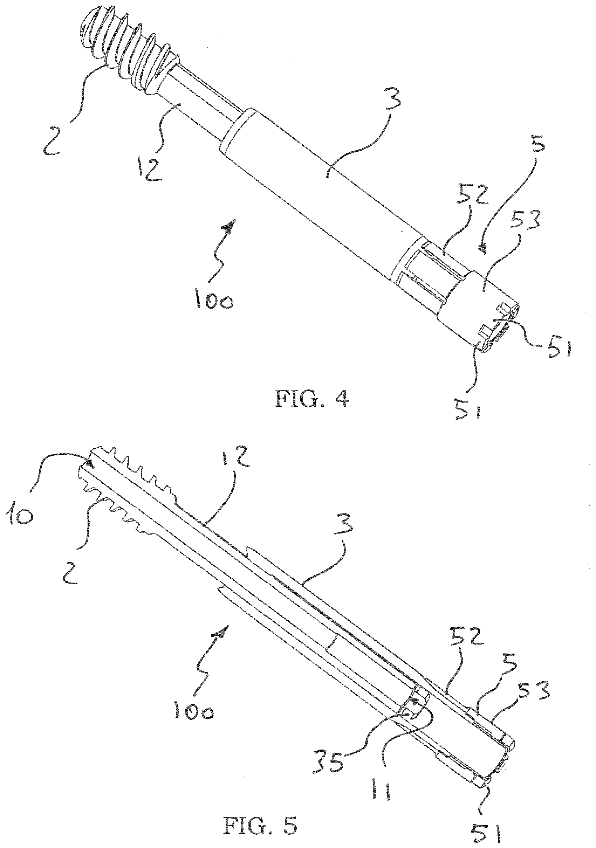

The endosseous screw assembly 100, which is shown in its entirety in Figure 4, comprises a longitudinal stem 1 and a connection sleeve 3.

The stem 1 and the connection sleeve 3 may be formed as one piece, so as to be included in a single body forming the endosseous screw assembly. In this case, no relative movement of the stem with respect to the connection sleeve is therefore envisaged.

Alternatively, the stem and the connection sleeve may be formed by two separate bodies with the aforementioned stem partially inserted inside the connection sleeve and movable with respect to the aforementioned connection sleeve.

The stem 1 of the endosseous screw assembly 100 comprises a threaded proximal portion 2 and an adjacent cylindrical portion 12.

In the example of embodiment described here, the entire stem 1 is channelled, namely has an axial passage which connects a distal opening 11 to a proximal opening 10. This solution allows the use of a guide wire in order to facilitate insertion of the endosseous screw assembly 100; moreover, the axial passage may advantageously define an access way for injecting *in situ* a bone substitute or other biological substances.

The threaded proximal portion 2 is designed to penetrate into the bone site of the patient and in particular is provided preferably with a self-boring tip which allows the screw to advance in the bone.

The tip has, formed in it, the proximal opening 10 of the aforementioned axial passage, while the distal opening 11 of the axial passage is present at the end of the cylindrical portion 12.

The connection sleeve 3 is formed by a hollow body inside which the stem is partly inserted and comprises first and second cylinders 32, 33 with different diameters which are connected by means of a conical portion 31. In particular, the first cylinder 32, in the proximal position, has a diameter greater than that of the second cylinder 33 such that the conicity of the conical portion 32 is directed in the distal direction, substantially away from the stem 1 of the endosseous screw assembly 100.

Preferably, the conical portion 31 of the connection sleeve has a conicity of between 8° and 12°, and preferably equal to 10°.

As already mentioned, if the connection sleeve is formed as a body separate from the stem, the aforementioned stem and connection sleeve are movable relative to each other.

In particular, the connection sleeve 3 is associated in a sliding relationship with the cylindrical portion 12 of the stem 1.

Sliding of the stem 1 is constrained axially in both directions using methods which are known in the sector.

Moreover, the relative rotation of the stem 1 and the connection sleeve 3 is prevented by suitable constraining means which are also known in the sector.

Essentially, the stem 1 may slide within certain limits inside the connection sleeve 3, but is rotationally constrained thereto.

According to the present invention, the cylindrical portion 12 of the stem 1 of the endosseous screw assembly 100 has second engaging means 35 which are designed to be joined together with a tool 101 for screwing the threaded proximal portion 2 into the bone.

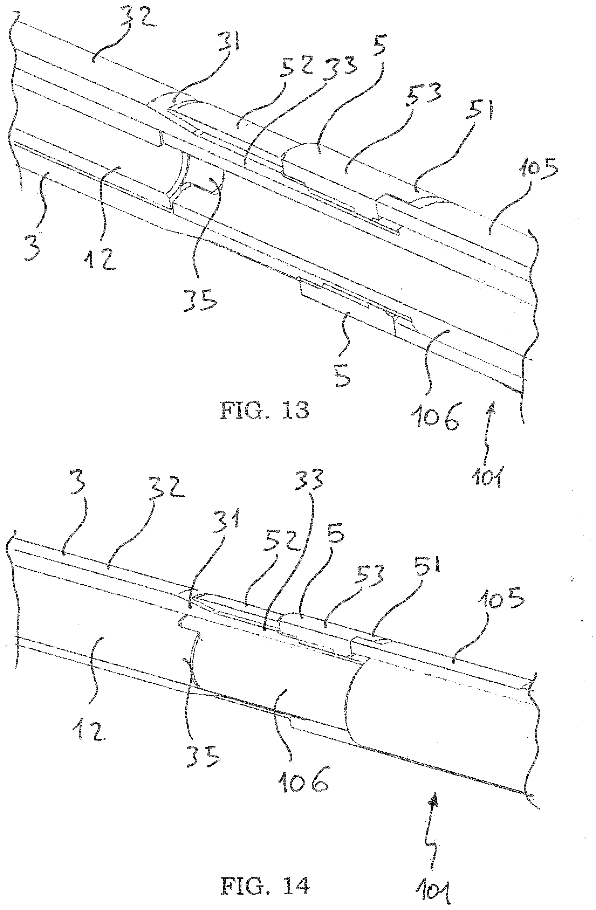

More specifically, as can be seen in Figures 13 and 14, the second engaging means 35 take the form of a toothing, in the example a pair of teeth, which project longitudinally from the distal end of the cylindrical portion 12 of the stem 1 and are shaped so as to reproduce the negative form of the tool for screwing the stem 1.

As will be explained more clearly below, the aforementioned tool 101 comprises substantially a first screwdriver 105 and a second screwdriver 106, the latter being able to joined together with the second engaging means 35.

The endosseous screw assembly 100, according to the invention, also comprises a ring nut which is screwed around the second cylinder 33 of the connection sleeve 3.

The ring nut 5 is formed by an annular body which has a distal end 53 having a smooth and continuous outer surface, a proximal end provided with deformable interference means 52, which will be described more fully below, and first engaging means formed in the example by teeth 51 which project longitudinally from the distal end 53 and are formed so as to cooperate with the first screwdriver 105 for screwing the ring nut 5 onto the connection sleeve 3.

The distal end 53 is internally threaded so as to be able to be screwed onto the second cylinder 33 of the connection sleeve 3.

The deformable interference means in the example shown take the form of a plurality of flexible fins 52 which extend longitudinally towards the proximal end from the distal end 53 of the annular body 5.

Basically, the proximal end of the annular body has longitudinal cuts which define the fins 52.

These fins 52 are connected to the distal end 53 only at their distal end. Since the ring nut 5 is made of flexible material, the fins 52 are able to flex, such that their free proximal end is able to move elastically in a substantially radial direction.

The fins 52, at their free proximal end, have a tapered internal surface so as to be able to slide along the conical portion 31 of the connection sleeve 3.

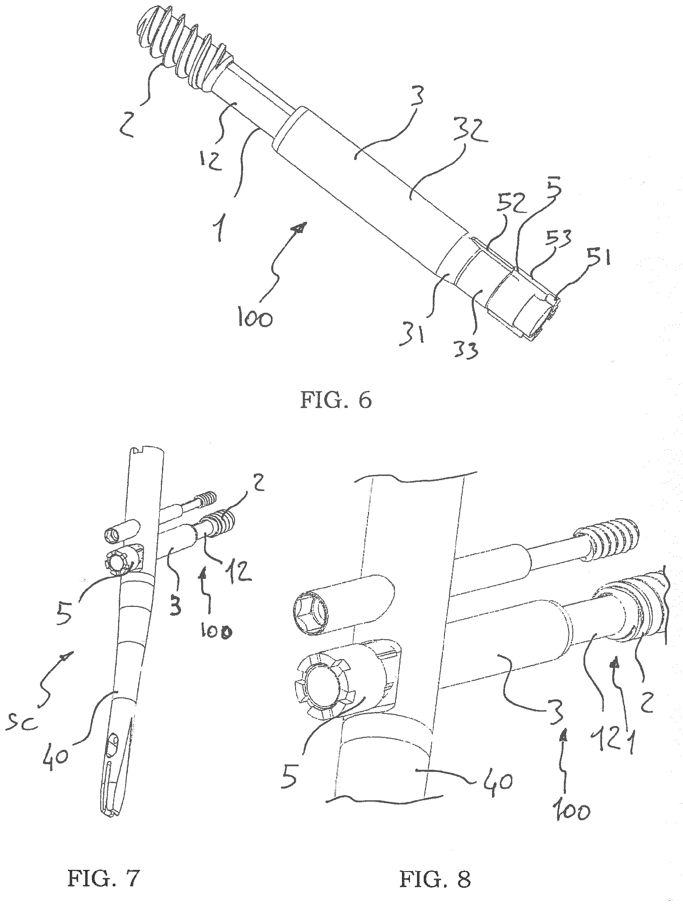

As can be seen in Figure 4, the endosseous screw assembly 100, before being inserted into the bone, has the ring nut 5 which is screwed onto the second cylinder 33 with the fins 52 which are positioned along a limited section of the conical portion 31. The larger outer diameter measured on the fins 52 which partially overlap the conical portion 31 does not exceed the outer diameter of the first cylinder 32, as can be seen in Figure 6.

Operationally speaking, locking of the internal fixation system is performed by inserting the endosseous screw assembly 100 inside the connection hole 42 and rotating the endosseous screw assembly 100 by means of the second screwdriver 106 which is engaged with the teeth 35 which project longitudinally from the distal end of the cylindrical portion 12 of the stem 1.

Basically, rotation of the stem 1 is performed, causing rotation of the threaded portion 2.

During screwing of the threaded portion 2, the connection sleeve 3 is also moved by the stem 1.

By operating the second screwdriver 106, the screw is screwed into the bone until it comes into contact with the nail 40.

Once contact is made, keeping the screw immobile with the second screwdriver 106, the first screwdriver 105 is rotated on the outside of the second screwdriver 106, thus operating the ring nut 5 with the fins 52.

The ring nut 5, which is threaded internally, rotates and advances on the connection sleeve 3 and the fins slide along the conical portion onto which they are widened and expanded.

Essentially, the stem 1 is kept fixed by means of the second screwdriver 106, while the ring nut 5 is made to rotate by means of the first screwdriver 105.

The presence of a thread in the connection between connection sleeve 3 and ring nut 5 causes, when the stem 1 is kept fixed, an advancing movement of the ring nut 5 axially in the proximal direction, i.e. towards the bone.

This relative axial movement of the sleeve 3 and the ring nut 5 is equal to a few tenths of a millimetre. This axial movement, in the proximal direction, of the ring nut 5, with the stem 1 kept locked, causes sliding of the fins 52 of the ring nut over the conical portion 31 of the connection sleeve 3, with a consequent radial displacement of said fins 52 which, sliding over the conical portion 31, tend to widen outwards.

This outwards widening of the fins 52 in turn causes frictional locking of the ring nut 5 and the internal wall 44 of the connection hole 42.

In other words, the fins 52 move over the conical portion 31 and, widening radially with respect to the initial position, push against the internal wall 44 of the connection hole 42, until they block rotation of said ring nut 5.

At this point the operation of screwing the ring nut 5 using the first screwdriver 105 is terminated.

Consequently the endosseous screw assembly is locked relative to the fixation member.

Obviously, the internal diameter of the connection hole 42 is chosen so as to allow the fins 52 to be snugly inserted when they rest on the connection sleeve 3, before being splayed.

Preferably, the internal diameter of the connection hole 42 is substantially equivalent to the diameter of the first cylinder 32, with the possibility of the latter sliding freely.

For a person skilled in the art it will be clear how both unscrewing of the ring nut 5 from the connection sleeve 3 and sliding of the ring nut 5 with respect to the internal wall 44 of the connection hole 42 are prevented, when the contact force exceeds a threshold value.

In other words the contact force present between the ring nut and the internal wall of the connection hole, in addition to preventing sliding of the endosseous screw assembly inside the connection hole, also results in a contact force between the ring nut and the connection sleeve, relative rotation of which is thus prevented.

As mentioned above, the tapered part of the internal surface of the fins 52 forms a receiving surface which facilitates sliding of the aforementioned fins along the conical portion 31 of the connection sleeve 3.

The stem and the connection sleeve are made of bio-compatible materials, preferably steel, titanium alloy or titanium. The ring nut, since it must ensure a sufficient elasticity for the deformable interference means, is made preferably of titanium.

As regards the tool 101 provided for screwing the stem and locking the endosseous screw assembly inside the connection hole, the first screwdriver 105 is formed as a hollow cylinder with a toothed end, having a diameter suitable for engagement with the first engaging means 51 of the ring nut 5.

The hollow cylinder forming the first screwdriver 105 houses, coaxially inside it, the second screwdriver 106 which is also cylindrical and suitable for engaging the second engaging means 35 included in the distal end of the cylindrical portion 12 of the stem 1.

An operator may rotate either one of the first and second screwdrivers independently, said first and second screwdrivers not being rotationally constrained.

In particular, the second screwdriver 106 may be rotated by means of an external handle 104, while the first screwdriver 105 is accessible manually by the operator, being arranged externally.

In order to provide a complete description of the invention in question, the method by means of which a surgeon can insert the endosseous screw assembly inside the fractured bone site and lock the endosseous screw assembly on the fixation member is now illustrated.

The implant of the fixation system, as described above, is performed by means of two consecutive steps, during which the surgeon first anchors the fixation member on the fractured bone site and then inserts the endosseous screw assembly through the patient's bone.

During a first anchoring step, therefore, the fixation member, whether it be a trochanteric nail or a plate, is oriented correctly and then fastened to the fractured bone of the patient.

Thereafter, the endosseous screw assembly is inserted inside the connection hole. This operation is performed with the aid of guiding means comprising a handle and a guide tube through which the endosseous screw assembly slides, pushed by the second screwdriver 106 defined above.

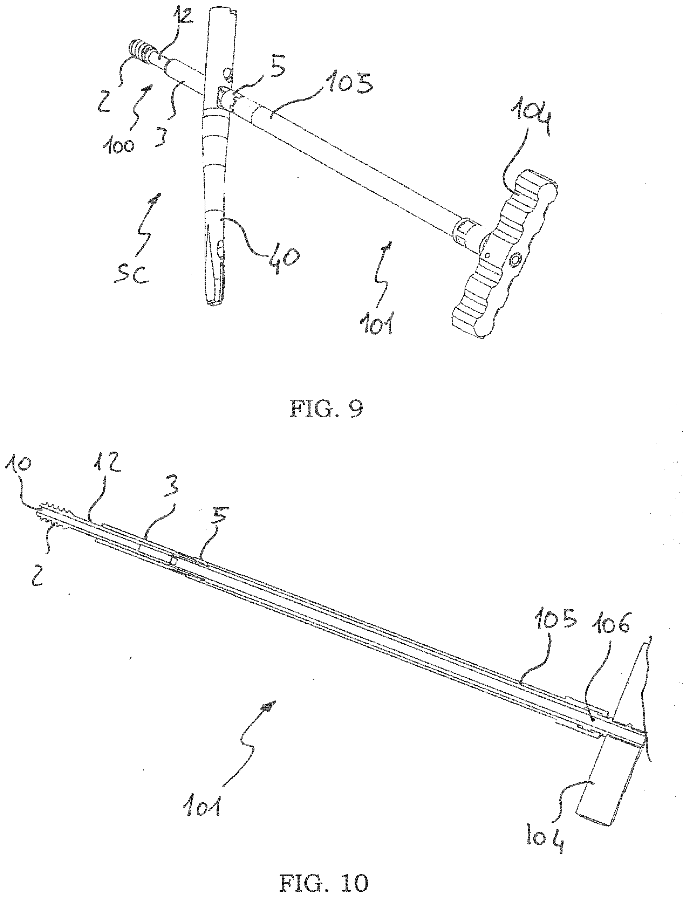

As can be seen from Figure 10, the second screwdriver 106 is joined together with the distal end of the cylindrical portion 12 of the stem 1 by means of the second engaging means 35, such that the surgeon may perform screwing of the threaded portion 2 inside the bone, when the endosseous screw assembly comes into contact with the bone tissue, exclusively by rotating the aforementioned second screwdriver 106 by means of the handle 104.

This operation of screwing the endosseous screw assembly continues until the ring nut 5 comes into contact against the fixation member.

At this point, once the step of advancing the threaded portion 2 inside the bone site has been terminated, the ring nut 5 is rotated by means of the first screwdriver 105, while the second screwdriver 106 which locks the stem 1 is kept fixed.

The threaded connection present between the ring nut 6 and the connection sleeve 3 therefore causes relative sliding of the two elements (connection member/ring nut) and in particular of the ring nut 5 along the second cylinder 33 of the connection sleeve 3. Screwing of the ring nut 5 on the connection sleeve 3, performed using the first screwdriver 105, causes sliding of the fins 52 along the conical portion 31 of the connection sleeve 3.

The conicity of the conical portion 31 cause the consequent radial flexing of the fins 52 outwards so as to push against the internal wall 44 of the connection hole.

A person skilled in the art will understand how this condition prevents both relative sliding of the ring nut with respect to the internal wall of the connection hole and rotation of the ring nut with respect to the connection sleeve, owing to the contact force present between the endosseous screw assembly and the fixation member inside the connection hole.

A person skilled in the art will understood how the endosseous screw assembly according to the invention allows simplification of the procedure for an implant of the corresponding fixation system, reducing both the number of tools and the operations required for the surgical operation.

It can be noted, in fact, how, after introduction of the endosseous screw assembly inside the guide tube, the surgeon may proceed using only the tool 101 comprising the two screwdrivers which are coaxial with each other.

Similarly, the operation of removing the endosseous screw assembly is facilitated, requiring again the use of only the tool 101.

In any case, even with the simplifications introduced, the fixation system will ensure correct supporting on the fracture site.

Advantageously, moreover, the duration of the surgical operations for the implant of such systems will be reduced.

Finally, since the surgeon must carry out a limited number of operations, a non-expert operator will also be able to perform the surgical operation.

Obviously a person skilled in the art, in order to satisfy any specific requirements which might arise, may make numerous modifications and variations to the invention described above, all of which may be contained moreover within the scope of protection of the invention, as defined by the following claims.

## Claims

1. Endosseous screw assembly (100) for an internal fixation system (SC), comprising:
a stem (1) extending longitudinally and having at least one threaded proximal portion (2) for allowing anchoring to a bone site of a patient;
a connection sleeve (3) suitable for introduction inside a connection hole (42) of a fixation member (40);
the stem and the connection sleeve being formed as one piece or by two separate bodies with the stem partially inserted inside the connection sleeve and movable with respect to the connection sleeve;
a ring nut (5) rotatably mounted on the connection sleeve (3) by means of a threaded connection, suitable for abutting against the connection hole (42) of the fixation member (40), and comprising first engaging means (51) suitable for engagement with a first screwdriver (105) for screwing the ring nut (5) on said connection sleeve (3);
said ring nut (5) further comprising a plurality of deformable interference means (52) which lie along a conical portion (31) of the connection sleeve (3); said conical portion (31) being designed to be seated inside the connection hole (42) when the connection sleeve (3) is introduced inside said connection hole (42) and being oriented so that relative sliding of the ring nut (5) and the connection sleeve (3), due to screwing of the ring nut (5) with respect to said connection sleeve (3), results in deformation of the deformable interference means (52) so as to obtain a contact force between the fixation member (40) and the ring nut (5) inside the connection hole (42) and lock the endosseous screw assembly (100) inside said connection hole (42) of the fixation member (40).

2. Endosseous screw assembly (100) for an internal fixation system (SC) according to the preceding claim 1, wherein the ring nut (5) comprises a distal ring (53), which is internally threaded and seats externally the first engaging means (51), and on a proximal end a plurality of flexible fins which form the deformable interference means (52).

3. Endosseous screw assembly (100) for an internal fixation system (SC) according to the preceding claim 2, wherein the flexible fins project from the distal ring (53) and extend longitudinally towards the stem (1) of the endosseous screw assembly (100), when the ring nut (5) is mounted on the connection sleeve (3).

4. Endosseous screw assembly (100) for an internal fixation system (SC) according to the preceding claims 2 or 3, wherein the flexible fins are tapered.

5. Endosseous screw assembly (100) for an internal fixation system (SC) according to any one of the preceding claims, wherein the connection sleeve (3) has a first cylinder (32) and a second cylinder (33) with different diameters, which are connected by means of the conical portion (31); the first cylinder having a greater diameter than the diameter of the second cylinder.

6. Endosseous screw assembly (100) for an internal fixation system (SC) according to any one of the preceding claims, wherein the conical portion (31) of the connection sleeve has a conicity of between 8° and 12°.

7. Endosseous screw assembly (100) for an internal fixation system (SC) according to any one of the preceding claims, wherein the stem (1) comprises a cylindrical distal portion (12) situated opposite the threaded proximal portion (2), said cylindrical distal portion (12) having second engaging means suitable for engagement with a second screwdriver (106) for screwing the proximal threaded portion (2) into the bone.

8. Endosseous screw assembly (100) for an internal fixation system (SDC) according to claim 7, wherein the second engaging means (35) comprises a toothing which projects longitudinally from the distal end of the cylindrical portion (12) of the stem (1) and are shaped so as to reproduce the negative form of the engaging means of the second screwdriver (106).

9. Endosseous screw assembly (100) for an internal fixation system (SC) according to any one of the preceding claims, wherein the stem (1) is rotationally constrained to the connection sleeve (3).

10. Fixation system (SC) comprising at least one endosseous screw assembly (1) according to one of the preceding claims, said internal fixation system comprising at least one fixation member (40) provided with at least one connection hole (42) suitable for receiving the connection sleeve (3) of said endosseous screw assembly (1).

11. Fixation system (SC) according to claim 10, wherein the at least one connection hole (42) has a flattened part (45) around the connection hole for receiving the ring nut (5) in contact therewith.

12. Fixation system (SC) according to claim 10 or 11, wherein a circumference defined by the deformable interference means (52) of the ring nut (5) has a diameter equivalent to the diameter of the connection hole (42).

13. Fixation system (SC) according to any one of claims 10-12, wherein the fixation member (40) is a trochanteric nail (40) or a plate.

14. Installation kit comprising a fixation system (SC) according to claims 10-13, a first screwdriver (105) and a second screwdriver (106), said first screwdriver (105), for screwing the ring nut (5) on the connection sleeve (3), being a hollow cylinder inside which the second screwdriver (106) is housed; said first and second screwdrivers (105; 106) being rotationally mobile relative to each other.

15. Installation kit according to claim 14, wherein the first screwdriver (105) is provided with an external handle (104).

## Patentansprüche

1. Enossale Schraubenanordnung (100) für ein inneres Fixierungssystem (SC), mit
einem Schaft (1), der sich der Länge nach erstreckt und mindestens einen mit einem Gewinde versehenen, proximalen Teil (2), um ein Verankern an einer Knochenstelle eines Patienten zuzulassen, hat;
einer Verbindungshülse (3), die zur Einführung ins Innere eines Verbindungsloches (42) eines Fixierungsteiles (40) geeignet ist;
wobei der Schaft und die Verbindungshülse als einzelnes Teil oder durch zwei getrennte Körper, wobei der Schaft teilweise in die Verbindungshülse eingeführt und relativ zu der Verbindungshülse beweglich ist, gebildet sind;
einer Ringmutter (5), die an der Verbindungshülse (3) mittels einer Gewindeverbindung drehbar angebracht ist, zum Anstoßen an dem Verbindungsloch (42) des Fixierungsteiles (40) geeignet ist und erste Eingriffsmittel (51) aufweist, die zum Eingriff mit einem ersten Schraubendreher (105) zum Schrauben der Ringmutter (5) auf die Verbindungshülse (3) geeignet sind;
wobei die Ringmutter (5) ferner eine Vielzahl von verformbaren Interferenzmitteln (52) aufweist, die an einem konischen Teil (31) der Verbindungshülse (3) entlang liegen; wobei der konische Teil (31) gestaltet ist, um im Innern des Verbindungsloches (3) zum Sitzen zu kommen, wenn die Verbindungshülse (3) ins Innere des Verbindungsloches (42) eingeführt ist, und ausgerichtet ist, so dass relatives Gleiten der Ringmutter (5) und der Verbindungshülse (3) infolge des Schraubens der Ringmutter (5) bezüglich der Verbindungshülse (3) in einer Verformung der verformbaren Interferenzmittel (52) resultiert, um eine Kontaktkraft zwischen dem Fixierungsteil (40) und der Ringmutter (5) im Innern des Verbindungsloches (42) zu erhalten und die enossale Schraubenanordnung (100) im Innern des Verbindungsloches (42) des Fixierungsteiles (40) zu verblocken.

2. Enossale Schraubenanordnung (100) für ein inneres Fixierungssystem (SC) nach dem vorhergehenden Anspruch 1, bei der die Ringmutter (5) einen distalen Ring (53) aufweist, der ein Innengewinde hat und an dem außen die ersten Eingriffsmittel (51) sitzen, und an einem proximalen Ende eine Vielzahl von biegsamen Lamellen aufweist, die die verformbaren Interferenzmittel (52) bilden.

3. Enossale Schraubenanordnung (100) für ein inneres Fixierungssystem (SC) nach dem vorhergehenden Anspruch 2, bei der die biegsamen Lamellen von dem distalen Ring (53) abstehen und der Länge nach auf den Schaft (1) der enossalen Schraubenanordnung (100) zu verlaufen, wenn die Ringmutter (5) an der Verbindungshülse (3) angebracht ist.

4. Enossale Schraubenanordnung (100) für ein inneres Fixierungssystem (SC) nach den vorhergehenden Ansprüchen 2 oder 3, bei der die biegsamen Lamellen verjüngt sind.

5. Enossale Schraubenanordnung (100) für ein inneres Fixierungssystem (SC) nach einem der vorhergehenden Ansprüche, bei der die Verbindungshülse (3) einen ersten Zylinder (32) und einen zweiten Zylinder (33) mit verschiedenen Durchmessern hat, die mittels des konischen Teiles (31) miteinander verbunden sind; wobei der erste Zylinder einen größeren Durchmesser als der Durchmesser des zweiten Zylinders hat.

6. Enossale Schraubenanordnung (100) für ein inneres Fixierungssystem (SC) nach einem der vorhergehenden Ansprüche, bei der der konische Teil (31) der Verbindungshülse eine Konizität zwischen 8° und 12° hat.

7. Enossale Schraubenanordnung (100) für ein inneres Fixierungssystem (SC) nach einem der vorhergehenden Ansprüche, bei der der Schaft (1) einen zylindrischen distalen Teil (12) aufweist, der gegenüber dem mit einem Gewinde versehenen proximalen Teil (2) angeordnet ist, wobei der zylindrische distale Teil (12) zweite Eingriffsmittel hat, die für den Eingriff mit einem zweiten Schraubendreher (106) zum Einschrauben des proximalen, mit einem Gewinde versehenen Teiles (2) in den Knochen geeignet sind.

8. Enossale Schraubenanordnung (100) für ein inneres Fixierungssystem (SDC) nach Anspruch 7, bei der die zweiten Eingriffsmittel (35) eine Verzahnung aufweisen, die der Länge nach von dem distalen Ende des zylindrischen Teiles (12) des Schaftes (1) absteht und so geformt ist, dass sie die negative Form der Eingriffsmittel des zweiten Schraubendrehers (106) wiedergibt.

9. Enossale Schraubenanordnung (100) für ein inneres Fixierungssystem (SC) nach einem der vorhergehenden Ansprüche, bei der der Schaft (1) am Drehen relativ zu der Verbindungshülse (3) gehindert ist.

10. Fixierungssystem (SC), das mindestens eine enossale Schraubenanordnung (1) nach einem der vorhergehenden Ansprüche aufweist, wobei das innere Fixierungssystem mindestens ein Fixierungsteil (40) aufweist, das mit mindestens einem Verbindungsloch (42) versehen ist, das zum Aufnehmen der Verbindungshülse (3) der enossalen Schraubenanordnung (1) geeignet ist.

11. Fixierungssystem (SC) nach Anspruch 10, bei dem das mindestens eine Verbindungsloch (42) einen abgeflachten Teil (45) um das Verbindungsloch herum hat, um die Ringmutter (5) in Kontakt damit aufzunehmen.

12. Fixierungssystem (SC) nach Anspruch 10 oder 11, bei dem ein von den verformbaren Interferenzmitteln (52) der Ringmutter (5) definierter Umkreis einen Durchmesser hat, der zu dem Durchmesser des Verbindungsloches (42) äquivalent ist.

13. Fixierungssystem (SC) nach einem der Ansprüche 10-12, bei dem das Fixierungsteil (40) ein Trochanternagel (40) oder eine Platte ist.

14. Einbausatz, der ein Fixierungssystem (SC) nach den Ansprüchen 10-13, einen ersten Schraubendreher (105) und einen zweiten Schraubendreher (106) aufweist, wobei der erste Schraubendreher (105), der zum Schrauben der Ringmutter (5) an die Verbindungshülse (3) dient, ein Hohlzylinder ist, in dem der zweite Schraubendreher (106) untergebracht ist; wobei der erste und der zweite Schraubendreher (105; 106) drehbeweglich relativ zueinander sind.

15. Einbausatz nach Anspruch 14, bei dem der erste Schraubendreher (105) mit einem äußeren Griff (104) versehen ist.

## Revendications

1. Ensemble vis endo-osseuses (100) pour un système de fixation interne (SC), comprenant :
une tige (1) s'étendant longitudinalement et présentant au moins une portion proximale filetée (2) pour permettre l'ancrage à un site osseux d'un patient,
un manchon de liaison (3) adapté à une introduction à l'intérieur d'un orifice de liaison (42) d'un élément de fixation (40),
la tige et le manchon de liaison étant formés d'une seule pièce ou de deux corps séparés avec la tige insérée en partie à l'intérieur du manchon de liaison et mobile par rapport au manchon de liaison,
un écrou à anneau (5) monté de manière rotative sur le manchon de liaison (3) à l'aide d'une liaison filetée, adapté à venir en butée contre l'orifice de liaison (42) de l'élément de fixation (40), et comprenant un premier moyen d'engagement (51) adapté à un engagement avec un premier tournevis (105) pour visser l'écrou à anneau (5) sur ledit manchon de liaison (3),
ledit écrou à anneau (5) comprenant en outre une pluralité de moyens d'interférence déformables (52) qui reposent le long d'une portion conique (31) du manchon de liaison (3), ladite portion conique (31) étant conçue pour être portée à l'intérieur de l'orifice de liaison (42) lorsque le manchon de liaison (3) est introduit à l'intérieur dudit orifice de liaison (42) et étant orientée de sorte qu'un coulissement relatif de l'écrou à anneau (5) et du manchon de liaison (3), du fait du vissage de l'écrou à anneau (5) par rapport audit manchon de liaison (3), résulte en une déformation des moyens d'interférence déformables (52) de manière à obtenir une force de contact entre l'élément de fixation (40) et l'écrou à anneau (5) à l'intérieur de l'orifice de liaison (42) pour verrouiller l'ensemble vis endo-osseuses (100) à l'intérieur dudit orifice de liaison (42) de l'élément de fixation (40).

2. Ensemble vis endo-osseuses (100) pour un système de fixation interne (SC) selon la revendication précédente 1, dans lequel l'écrou à anneau (5) comprend un anneau distal (53), qui est fileté de manière interne et porte de manière externe le premier moyen d'engagement (51), et sur une extrémité proximale une pluralité d'ailettes flexibles qui forment les moyens d'interférence déformables (52).

3. Ensemble vis endo-osseuses (100) pour un système de fixation interne (SC) selon la revendication précédente 2, dans lequel les ailettes flexibles font saillie à partir de l'anneau distal (53) et s'étendent longitudinalement vers la tige (1) de l'ensemble vis endo-osseuses (100), lorsque l'écrou à anneau (5) est monté sur le manchon de liaison (3).

4. Ensemble vis endo-osseuses (100) pour un système de fixation interne (SC) selon les revendications précédentes 2 ou 3, dans lequel les ailettes flexibles sont effilées.

5. Ensemble vis endo-osseuses (100) pour un système de fixation interne (SC) selon une quelconque des revendications précédentes, dans lequel le manchon de liaison (3) présente un premier cylindre (32) et un second cylindre (33) de diamètres différents, qui sont reliés à l'aide de la portion conique (31), le premier cylindre présentant un diamètre supérieur au diamètre du second cylindre.

6. Ensemble vis endo-osseuses (100) pour un système de fixation interne (SC) selon une quelconque des revendications précédentes, dans lequel la portion conique (31) du manchon de liaison présente une conicité comprise entre 8° et 12°.

7. Ensemble vis endo-osseuses (100) pour un système de fixation interne (SC) selon une quelconque des revendications précédentes, dans lequel la tige (1) comprend une portion distale cylindrique (12) située de manière opposée à la portion proximale filetée (2), ladite portion distale cylindrique (12) présentant un second moyen d'engagement adapté à un engagement avec un second tournevis (106) pour viser la portion filetée proximale (2) dans l'os.

8. Ensemble vis endo-osseuses (100) pour un système de fixation interne (SDC) selon la revendication 7, dans lequel le second moyen d'engagement (35) comprend des dents qui font saillie longitudinalement à partir de l'extrémité distale de la portion cylindrique (12) de la tige (1) et sont façonnées de manière à reproduire la forme négative du moyen d'engagement du second tournevis (106).

9. Ensemble vis endo-osseuses (100) pour un système de fixation interne (SC) selon une quelconque des revendications précédentes, dans lequel la tige (1) est contrainte en rotation vers le manchon de liaison (3).

10. Système de fixation (SC) comprenant au moins un ensemble vis endo-osseuses (1) selon une des revendications précédentes, ledit système de fixation interne comprenant au moins un élément de fixation (40) doté d'au moins un orifice de liaison (42) adapté à recevoir le manchon de liaison (3) dudit ensemble vis endo-osseuses (1).

11. Système de fixation (SC) selon la revendication 10, dans lequel l'au moins un orifice de liaison (42) présente une partie aplatie (45) autour de l'orifice de liaison pour recevoir l'écrou à anneau (5) en contact avec celle-ci.

12. Système de fixation (SC) selon la revendication 10 ou 11, dans lequel une circonférence définie par les moyens d'interférence déformables (52) de l'écrou à anneau (5) présente un diamètre équivalent au diamètre de l'orifice de liaison (42).

13. Système de fixation (SC) selon une quelconque des revendications 10 à 12, dans lequel l'élément de fixation (40) peut être un clou trochantérien (40) ou une plaque.

14. Kit d'installation comprenant un système de fixation (SC) selon les revendications 10 à 13, un premier tournevis (105) et un second tournevis (106), ledit premier tournevis (105), pour visser l'écrou à anneau (5) sur le manchon de liaison (3), étant un cylindre creux à l'intérieur duquel le second tournevis (106) est logé, lesdits premier et second tournevis (105, 106) étant mobiles en rotation l'un par rapport à l'autre.

15. Kit d'installation selon la revendication 14, dans lequel le premier tournevis (105) est doté d'une poignée externe (104).
